# DEMANDE DE BREVET EUROPEEN

(11) **EP 3 290 873 A1**
(43) Date de publication de la demande: **07.03.2018**
(21) Numéro de dépôt: 17306014.6
(22) Date de dépôt: 28.07.2017
(51) Int. Cl.: G01F 1/32, A61M 16/00, A61B 5/083

(54) **DÉBITMÈTRE À OSCILLATION FLUIDIQUE À ORIFICES DE MESURE SYMÉTRIQUES POUR DISPOSITIF DE D'OBSERVANCE D'UN TRAITEMENT D'OXYGÉNOTHÉRAPIE**

(30) Priorité: 02.09.2016 FR 1658167
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: AMMOURI, Fouad, 91300 MASSY (FR)
(74) Mandataire: Pittis, Olivier

(57) **Abrégé**

L'invention concerne un débitmètre à oscillation fluidique comprenant une chambre de stabilisation (1) comprenant un élément stabilisateur de flux (11), une chambre d'oscillation (2) comprenant un élément à reflux (21) configuré pour créer au moins un tourbillon gazeux oscillant dans la chambre d'oscillation (2), ledit élément à reflux (21) étant compris entre deux parois parallèles (28, 29) délimitant la chambre d'oscillation (2), un conduit de liaison (3) reliant fluidiquement la chambre de stabilisation (1) à la chambre d'oscillation (2), et un plan de symétrie (P) séparant le conduit de liaison (3), la chambre de stabilisation (1), l'élément stabilisateur de flux (11), la chambre d'oscillation fluidique (2) et l'élément à reflux (21) en deux parties égales et symétriques par rapport audit plan de symétrie (P). L'une des deux parois parallèles (28, 29) comprend deux orifices de mesure (24, 25) agencés symétriquement par rapport au plan de symétrie (P), et le conduit de liaison (3) a une section rectangulaire. Dispositif de suivi ou d'observance d'un traitement d'oxygénothérapie comprenant un tel débitmètre à oscillation fluidique et installation d'oxygénothérapie comprenant une source de gaz respiratoire, une interface de distribution de gaz et un tel dispositif de suivi ou d'observance.

## Description

La présente invention concerne un débitmètre à oscillation fluidique utilisable en oxygénothérapie, et un dispositif de suivi ou d'observance d'un traitement d'oxygénothérapie équipé d'un tel débitmètre à oscillation fluidique, et installation d'oxygénothérapie associée.

Dans le cadre d'un traitement par oxygénothérapie d'un patient à domicile, on utilise habituellement un dispositif de suivi ou d'observance, venant s'insérer entre la source de gaz, typiquement une source d'oxygène, et le patient, de manière à permettre de suivre les consommations d'oxygène par le patient et s'assurer ainsi que celui-ci observe bien son traitement. Un tel dispositif peut être équipé d'un module de communication permettant de transmettre les données à distance, par exemple à un serveur distant.

Ainsi, WO-A-2009/136101 décrit un dispositif de suivi de l'oxygénothérapie chez un patient soigné à son domicile par administration d'oxygène comprenant un boitier traversé par un conduit, un ou plusieurs capteurs de pression, un microprocesseur, une mémoire, une batterie d'alimentation en courant électrique et une antenne radiofréquence.

EP-A-2670463 propose un dispositif analogue incluant en outre un accéléromètre permettant de suivre les besoins variables en oxygène du patient en fonction de son activité physique, en particulier activité normale ou soutenue, faible activité ou repos, ou sommeil par exemple.

Par ailleurs, EP-A-2506766 enseigne un dispositif permettant de suivre la respiration d'un patient comprenant un capteur de pression différentiel agencé sur un conduit de gaz comprenant en outre une configuration interne de type venturi. Ce dispositif est principalement dédié à la détection des apnées ou hypopnées chez un patient traité sous pression positive continue.

En outre, EP-A-2017586 propose un dispositif permettant de suivre la respiration du patient en ventilation normale ou sous pression positive continue. Il comprend un conduit de gaz équipé d'un élément réducteur de diamètre engendrant une perte de charge et un capteur de pression différentielle permettant de déterminer la pression et le débit du gaz.

Or, ces dispositifs connus ne sont pas idéaux car ils sont, selon le cas, encombrants, fortement consommateurs d'énergie, sensibles à la dérive des capteurs, imprécis pour estimer le volume de gaz fourni au patient, notamment pour les débits faibles, ou encore sensibles aux variations de pression de la source...

Au vu de cela, le problème qui se pose est de permettre d'améliorer la détermination du débit du gaz administré au patient et qui soit de préférence de faible consommation électrique, de faible encombrement, c'est-à-dire miniaturisé et peu couteux.

La solution de l'invention est alors un débitmètre à oscillation fluidique utilisable en oxygénothérapie, en particulier destiné à équiper un dispositif d'observance de patient, comprenant :
- une chambre de stabilisation comprenant un élément stabilisateur de flux,
- une chambre d'oscillation comprenant un élément à reflux configuré pour créer au moins un tourbillon gazeux oscillant (ou « vortex ») dans la chambre d'oscillation, l'élément à reflux étant compris entre deux parois parallèles délimitant la chambre d'oscillation,
- un conduit de liaison reliant fluidiquement la chambre de stabilisation à la chambre d'oscillation, et
- un plan de symétrie P séparant le conduit de liaison, la chambre de stabilisation, l'élément stabilisateur de flux, la chambre d'oscillation fluidique et l'élément à reflux en deux parties égales et symétriques par rapport audit plan de symétrie P,
caractérisé en ce que :
- l'une desdites deux parois parallèles délimitant la chambre d'oscillation comprend deux orifices de mesure agencés symétriquement par rapport au plan de symétrie P et sur un axe perpendiculaire au plan de symétrie P, et séparés l'un de l'autre d'une distance d comprise entre 0,5 mm et 15 mm, et
- le conduit de liaison a une section rectangulaire de largeur lₒ et de hauteur hₒ telles que : 6,5.lₒ ≥ hₒ ≥ 3.lₒ, c'est-à-dire que la hauteur hₒ est supérieure ou égale à 3 fois la largeur lₒ du conduit de liaison mais inférieure ou égale à 6,5 fois la largeur lₒ du conduit de liaison.

Le fait de choisir une limitation supérieure de la profondeur hₒ du conduit de liaison à 6,5 fois la largeur lₒ est liée, en premier lieu, à l'existence d'une limitation minimale de vitesse dans le conduit de liaison à partir de laquelle les oscillations de pression commencent à s'opérer dans la chambre d'oscillation. D'autre part, pour un débit donné, plus la hauteur hₒ de conduit de liaison est importante et plus la vitesse à l'intérieur est faible. Par conséquent, l'amplitude de variation de la pression au niveau des capteurs (qui est proportionnelle à la vitesse dans le conduit de liaison au carré) devient de plus en plus faible et la mesure de plus en plus sensible au bruit. Dans ces conditions, la qualité de la mesure aura donc tendance à se détériorer. Il convient donc de limiter la hauteur hₒ aux valeurs susmentionnées pour éviter ou minimiser ce phénomène.

Selon le cas, l'invention peut comprendre l'une ou plusieurs des caractéristiques techniques suivantes :
- de préférence, le conduit de liaison a une section rectangulaire de largeur lₒ et de hauteur hₒ, telles que : 6.lₒ ≥ hₒ.
- de préférence, le conduit de liaison a une section rectangulaire de largeur lₒ et de hauteur hₒ, telles que : 5,5.lₒ ≥ hₒ.
- de préférence, le conduit de liaison a une section rectangulaire de largeur lₒ et de hauteur hₒ, telles que : hₒ ≥ 3,1.lₒ.
- de préférence, le conduit de liaison a une section rectangulaire de largeur lₒ et de hauteur hₒ, telles que : hₒ ≥ 3,5.lₒ.
- le conduit de liaison de section rectangulaire a une longueur Lₒ telle que 2.lₒ ≤ Lₒ ≤ 15.lₒ.
- les deux orifices de mesure sont séparés d'une distance d comprise entre 0,5 mm et 10 mm, de préférence encore entre 1 mm et 6 mm.
- le gaz est de l'air, de l'oxygène ou un mélange air/oxygène.
- la chambre d'oscillation comprend une paroi périphérique reliant les deux parois parallèles, c'est-à-dire les deux parois agencées en vis-à-vis ou face à face dont l'une porte les deux orifices de mesure.
- les deux parois parallèles délimitant la chambre d'oscillation forment le plafond et le sol de la chambre d'oscillation, c'est-à-dire que les deux orifices de mesure sont agencés dans le sol ou le plafond.
- l'élément stabilisateur de flux est configuré pour permettre de rendre le profil de vitesse du gaz en sortie de cet élément en deux dimensions (2D), donc invariable dans la direction perpendiculaire au plan du débitmètre, et de plus symétrique par rapport au plan de symétrie. En effet, le profil de vitesse du gaz arrivant en entrée de cet élément est souvent en 3 dimensions (3D) et dissymétrique. Le fait de changer brutalement la direction de l'écoulement en entrée de cet élément dans une section rectangulaire en plus qui se rétrécit au fur et à mesure qu'on s'approche du conduit de liaison, qui est lui-même de section rectangulaire, permet de transformer l'écoulement 3D en écoulement 2D. D'autre part, la géométrie symétrique par rapport au plan de symétrie de cet élément permet de symétriser aussi le profil de vitesse.
- le conduit de liaison convoie le gaz de la chambre de stabilisation à la chambre d'oscillation en accélérant la vitesse du gaz car la section rectangulaire de passage du gaz est inférieure à celle du passage agencé dans l'élément stabilisateur. En effet, il faut une vitesse de gaz supérieure à une valeur minimale en entrée de la chambre d'oscillateur pour déclencher les oscillations car, en l'absence de vitesse minimale, mesurer le débit de gaz n'est pas possible.
- les deux orifices de mesure sont fermés, c'est-à-dire recouverts, par une membrane fluidiquement étanche. Cette membrane transmet les variations de pression du côté de la chambre d'oscillation vers l'endroit où se trouvent les capteurs, c'est-à-dire microphones ou capteurs de pression.
- l'élément à reflux comprend une partie de section semi-cylindrique agencée face au conduit de liaison.
- la chambre de stabilisation comprenant un premier orifice d'entrée et un premier orifice de sortie agencés sur le plan de symétrie P. Le gaz entre dans la chambre de stabilisation par le premier orifice d'entrée et ressort de la chambre de stabilisation par le premier orifice de sortie.
- la chambre d'oscillation comprenant un second orifice d'entrée et un second orifice de sortie agencés sur le plan de symétrie P. Le gaz entre dans la chambre d'oscillation par le second orifice d'entrée et ressort de la chambre d'oscillation par le second orifice de sortie.
- le conduit de liaison relie fluidiquement le premier orifice de sortie de la chambre de stabilisation au second orifice d'entrée de la chambre d'oscillation.
- il comprend en outre un ou plusieurs capteurs de pression ou microphones raccordés auxdits deux orifices de mesure de manière à permettre de mesurer la pression dans la chambre d'oscillation, de préférence des microphones.
- chaque orifice de mesure est relié à un capteur de pression ou à un microphone.
- un canal d'entrée est relié fluidiquement au premier orifice d'entrée de la chambre de stabilisation. Le canal d'entrée alimente la chambre de stabilisation en gaz.
- il comprend en outre un boitier au sein duquel sont agencés le conduit de liaison, la chambre de stabilisation, l'élément stabilisateur de flux, la chambre d'oscillation fluidique, l'élément à reflux et le ou les capteurs de pression ou microphones.
- l'élément stabilisateur de flux est espacé de la paroi périphérique de la chambre de stabilisation de manière à créer des passages pour le gaz autour dudit élément stabilisateur de flux. Le flux de gaz traverse donc la chambre de stabilisation en contournant l'élément stabilisateur de flux, c'est-à-dire en passant de part et d'autre de l'élément stabilisateur de flux.
- un conduit d'évacuation de gaz est en communication fluidique avec le second orifice de sortie de gaz de la chambre d'oscillation de manière à récupérer le gaz sortant de la chambre d'oscillation.

L'invention concerne un dispositif de suivi ou d'observance d'un traitement d'oxygénothérapie comprenant un débitmètre à oscillation fluidique selon l'invention.

Par ailleurs, l'invention concerne aussi une installation d'oxygénothérapie comprenant :
- une source de gaz respiratoire, par exemple un appareil de distribution de gaz ou une bouteille de gaz,
- une interface de distribution de gaz permettant de distribuer le gaz respiratoire à un patient, tel que des canules nasales ou un masque respiratoire, et
- un dispositif de suivi ou d'observance à débitmètre à oscillation fluidique selon l'invention.

L'invention va maintenant être mieux comprise grâce à la description détaillée suivante, faite à titre illustratif mais non limitatif, en référence aux Figures annexées parmi lesquelles :
- la Figure 1 est un schéma du principe de fonctionnement d'un débitmètre à oscillation fluidique selon l'invention,
- la Figure 2 est une représentation tridimensionnelle d'un débitmètre à oscillation fluidique selon l'invention analogue à celui de la Figure 1,
- la Figure 3 est un schéma de principe d'un mode de réalisation d'un dispositif de suivi ou d'observance d'un traitement d'oxygénothérapie comprenant un débitmètre à oscillation fluidique selon l'invention,
- la Figure 4 représente une installation d'oxygénothérapie incluant un dispositif de suivi ou d'observance selon la Figure 3 un débitmètre à oscillation fluidique selon l'invention,
- la Figure 5 montre l'emplacement des capteurs et la géométrie testés lors des essais de simulation,
- les Figures 6 à 12 sont des représentations graphiques des essais de simulation réalisés pour montrer l'importance du positionnement précis des orifices de mesure, et
- la Figure 13 schématise le conduit de liaison de section rectangulaire d'un débitmètre à oscillation fluidique selon l'invention.

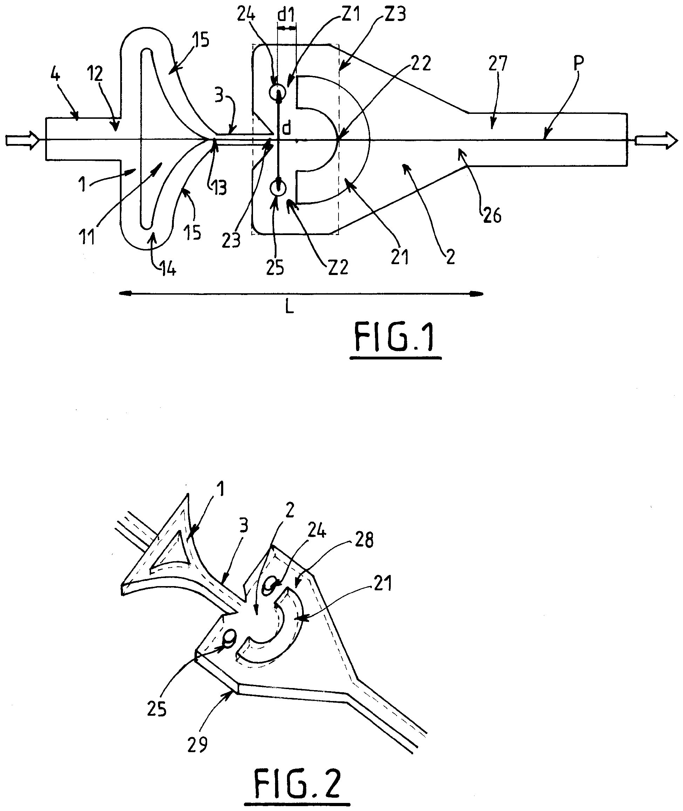

La Figure 1 est un schéma du principe de fonctionnement d'un débitmètre à oscillation fluidique (vue de dessus) selon l'invention. Il comprend une chambre de stabilisation 1 dans laquelle est agencé un élément stabilisateur de flux 11, ayant ici une section triangulaire, et une chambre d'oscillation 2 comprenant un élément à reflux 21 ayant une forme de demi-cylindre, lequel est configuré en arc de cercle 22 pour créer un tourbillon ou vortex gazeux oscillant. Le tourbillon oscille en fait entre deux zones Z1, Z2 situées schématiquement au niveau des extrémités du demi-cylindre formant l'élément à reflux 21.

L'élément à reflux 21 est pris en sandwich entre deux parois parallèles 28, 29 délimitant la chambre d'oscillation 2 en haut et en bas respectivement (Figure 2), c'est-à-dire formant le plafond et le sol de la chambre d'oscillation 2.

Un conduit de liaison 3 relie fluidiquement la chambre de stabilisation 1 à la chambre d'oscillation 2 de sorte que le gaz qui entre dans la chambre de stabilisation 1, la traverse et alimente ensuite la chambre d'oscillation 2. Le conduit de liaison 3 y débouche en vis-à-vis, c'est-à-dire en face ou en regard, de l'élément à reflux 21 de forme semi-cylindrique, ce qui engendre une oscillation du flux et une formation de tourbillons dans les deux zones Z1 et Z2 suscitées.

Comme on le voit, il existe en fait un plan de symétrie P séparant l'ensemble du système, en particulier le conduit de liaison 3, la chambre de stabilisation 1, l'élément stabilisateur de flux 11, la chambre d'oscillation fluidique 2 et l'élément à reflux 21, en deux parties égales et symétriques par rapport à ce plan de symétrie P.

Une telle configuration est connue et décrite dans la publication : *Yves Le Guer ; Jet confiné, dispersions fluide-particules et mélange chaotique ; Engineering Sciences ; Université de Pau et des Pays de l'Adour; 2005,* et dans le document WO 93/22627. Selon ce document, la profondeur du canal alimentant la chambre d'oscillation est de 70 mm et sa largeur de 10 mm.

Toutefois, une telle architecture n'est pas suffisante, notamment si le positionnement des prises de pression du débitmètre n'est pas choisi avec soin. En effet, si les prises de mesures de pression sont mal positionnées, un débitmètre équipé d'un tel système ne sera pas assez performant.

En d'autres termes, il a été constaté que certains dimensionnements sont particulièrement importants et doivent être respectés pour obtenir un débitmètre efficace et précis, en particulier le positionnement des prises de pression et les dimensions (hₒ, lₒ) du conduit de liaison 3 reliant fluidiquement la chambre de stabilisation 1 à la chambre d'oscillation 2.

Ainsi, il a été mis en évidence, lors d'essais opérés dans le cadre de la présente invention, que, pour assurer une mesure efficace de la variation en fonction du temps de la pression du gaz au sein de la chambre à reflux 2 dans laquelle oscille le flux gazeux en formant des tourbillons gazeux dans les zones Z1, Z2, il convient de choisir avec précision l'emplacement du site de mesure des capteurs de pression ou des microphones, à savoir les deux orifices de mesure 24, 25 auxquels sont reliés les capteurs de pression ou microphones (non représentés).

Selon l'invention, les deux orifices de mesure 24, 25 doivent être agencés, dans le plafond 28 (ou dans le sol 29) de la chambre à reflux 2, c'est-à-dire approximativement au-dessus des zones Z1, Z2 où se forment les tourbillons, et surtout symétriquement par rapport au plan de symétrie P du débitmètre en respectant impérativement entre eux, une distance d (mesurée entre les axes ou centres des orifices de mesures comprise entre 0,5 et 15 mm (cf. Figure 1), de préférence entre 0,5 et 10 mm, par exemple de l'ordre de 1 à 6 mm.

Les deux orifices de mesure 24, 25, reliés de préférence à des microphones, se situent sur un axe perpendiculaire au plan de symétrie P, et de façon préférentielle dans la zone Z3 représentée en pointillés sur la Figure 1, comme expliqué ci-après.

En fait, le principe d'un débitmètre à oscillation fluidique tel que décrit dans les publications susmentionnées ne permet pas de réaliser un débitmètre performant si le positionnement des deux orifices de mesure 24, 25 n'est pas choisi avec soin.

En effet, le positionnement des deux orifices de mesure 24, 25, l'un par rapport à l'autre, ainsi que par rapport à d'autres éléments de la géométrie du système de débitmètre joue un rôle important dans la perception de la fréquence d'oscillation de la pression du tourbillon et par conséquent influencent la précision de calcul du débit à partir des valeurs de pression mesurées par ces capteurs.

Ainsi, il est aussi indispensable de dimensionner de façon particulière le conduit de liaison 3 qui amène le flux de gaz dans la chambre de à reflux 2 où se trouvent les deux orifices de mesure 24, 25 reliés préférentiellement à des microphones (non montrés), comme expliqué ci-après.

Il est à souligner que les deux orifices de mesure 24, 25 sont préférentiellement fermés par une membrane fluidiquement étanche de manière à assurer le bon fonctionnement des microphones. En fait, la pression dans la chambre d'oscillation 2 se transmet aux capteurs ou aux microphones, via les deux orifices 24, 25, et au travers des membranes qui recouvrent ces deux orifices 24, 25. De préférence, la membrane a une épaisseur très fine au niveau des capteurs 24 et 25, typiquement de l'ordre de 50 à 500 µm environ; ailleurs, son épaisseur peut être comprise entre 1 et 2 mm, voire plus.

En fait, le flux de gaz circule dans le sens des flèches (=>) représentées en Figure 1. Le flux de gaz, par exemple de l'oxygène ou de l'air enrichi en oxygène, arrive par un canal d'entrée 4 est relié fluidiquement au premier orifice d'entrée 12 de la chambre de stabilisation 1 et pénètre dans ladite chambre de stabilisation 1, via ce premier orifice d'entrée 12.

Au sein de la chambre de stabilisation 1, le flux est soumis à une stabilisation par l'élément stabilisateur de flux 11, qui est de section se rapprochant de celle triangulaire avec sa base orientée en vis-à-vis du débouché du canal d'entrée 4, donc en face du premier orifice d'entrée 12. En fait, la section de l'élément stabilisateur de flux 11 est légèrement concave en se rapprochant de plus en plus de l'entrée 13 du conduit 3.

Le flux gazeux contourne donc l'élément stabilisateur de flux 11 en passant dans des passages 15 aménagés de part et d'autre de celui-ci. Les passages 15 sont en fait délimités par la surface externe de l'élément stabilisateur de flux 11 et par la paroi périphérique interne 14 de la chambre de stabilisation 1. En d'autres termes, l'élément stabilisateur de flux 11 est espacé de la paroi périphérique 14 de la chambre de stabilisation 1 de manière à créer des passages 15 pour le gaz autour dudit élément stabilisateur de flux 11.

Le flux gazeux ressort ensuite de la chambre de stabilisation 1 par le premier orifice de sortie 13 et est acheminé par le conduit de liaison 3 qui relie fluidiquement le premier orifice de sortie 13 de la chambre de stabilisation 1 au second orifice d'entrée 23 de la chambre d'oscillation 2.

Les premier et second orifices d'entrée 12, 23 et les premier et second orifices de sortie 13, 26 sont agencés de façon symétrique par rapport au plan de symétrie P, comme visible sur la Figure 1.

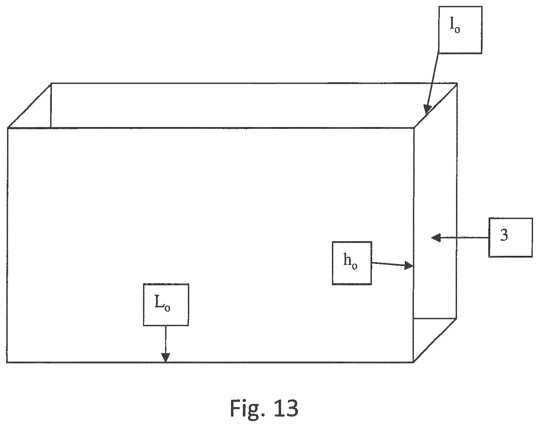

Selon la présente invention, afin de pouvoir assurer des mesures efficaces, il convient aussi de configurer et dimensionner le conduit de liaison 3 de manière particulière. Ainsi, selon l'invention, le conduit de liaison 3 est de section rectangulaire, c'est-à-dire qu'il est de forme générale parallélépipédique, avec une largeur lₒ et une hauteur hₒ telles que : 6,5.lₒ ≥ hₒ ≥ 3.lₒ, où la largeur lₒ est par exemple de 0,5 à 1,5 mm, de préférence encore entre 0,8 et 1,3 mm; ceci est illustré en Figure 13. Avantageusement, on choisit hₒ et lₒ, tels que : hₒ ≥ 3,1.lₒ, de préférence encore : hₒ ≥ 3,5.lₒ et/ou 6.lₒ ≥ hₒ.

En d'autres termes, choisir un conduit de liaison 3 dont la largeur sera faible/petite par rapport à sa hauteur va permettre d'obtenir un flux laminaire en 2D et à vitesse suffisamment élevée, ce qui va favoriser son oscillation dans la chambre de reflux 2.

Par ailleurs, il convient aussi préférentiellement de veiller à respecter une longueur Lₒ du conduit de liaison 3 par rapport à sa largeur lₒ, telle que 2.lₒ ≤ Lₒ ≤ 10.lₒ, avec préférentiellement : 3.lₒ≤Lₒ≤7.lₒ.

D'une façon générale, comme illustré en Figure 1, le flux pénètre alors dans la chambre d'oscillation 2 et vient y impacter l'élément à reflux 21 de forme semi-cylindrique, ce qui crée le tourbillon oscillant entre les deux zones Z1 et Z2, comme expliqué ci-avant.

Le gaz continue ensuite sa course dans la chambre d'oscillation 2 avant d'en ressortir par un conduit d'évacuation de gaz 27 qui est en communication fluidique avec le second orifice de sortie 26 de gaz de la chambre d'oscillation 2.

On comprend donc qu'à partir d'un champ de vitesse symétrique en 2 dimensions, on crée un tourbillon dont la localisation (zones Z1 et Z2) va osciller avec une fréquence proportionnelle à la valeur du débit du fluide qui y circule. En plaçant des microphones ou des organes/capteurs de mesure de pression en dehors du conduit du fluide, c'est-à-dire au dessus des zones Z1, Z2 où se forment les tourbillons (i.e. les vortex), on peut mesurer la présence ou non d'une dépression du gaz.

Le débitmètre de l'invention permet de déterminer de manière non-intrusive, miniaturisée, peu coûteuse et avec une perte de charge limitée par rapport à un débitmètre à organe déprimogène, le débit de gaz qui y circule.

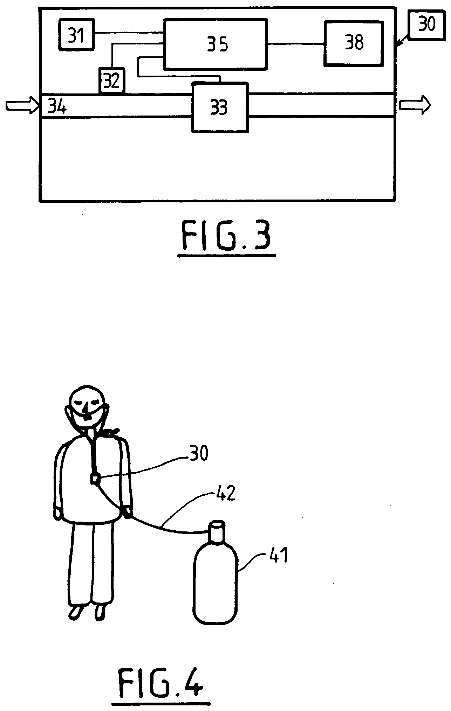

L'ensemble du système est compris dans un boitier 30 visible en Figure 3, en particulier le conduit de liaison 3, la chambre de stabilisation 1, l'élément stabilisateur de flux 11, la chambre d'oscillation fluidique 2, l'élément à reflux 21 et le ou les capteurs de pression ou microphones.

Par ailleurs, des moyens de pilotage 35, telle une carte électronique à microprocesseur, par exemple un microcontrôleur, est reliée électriquement aux capteurs de pression ou microphones de manière à recueillir et exploiter les mesures de pression en extrayant leur fréquence d'oscillation et ensuite en déduire un débit de gaz, comme illustré en Figure 3 et expliqué ci-après.

La Figure 2 est une représentation tridimensionnelle du débitmètre de la Figure 1 permettant de visualiser la localisation des orifices de mesure 24, 25 dans le plafond 28 de la chambre de reflux 2.

La Figure 3 est un schéma de principe d'un mode de réalisation d'un dispositif de suivi ou d'observance d'un traitement d'oxygénothérapie comprenant un débitmètre 33 à oscillation fluidique selon l'invention, comprenant un boitier 30 incorporant un premier capteur de pression absolue 31 pour mesurer la pression ambiante, c'est-à-dire la pression atmosphérique, et un second capteur de pression absolue 32 pour mesurer la pression absolue dans la canule 34, lequel est placé en contact direct avec la canule 34, avant ou après le débitmètre 33 à oscillation fluidique selon l'invention. Le flux de gaz circule dans la canule ou conduit 34, dans le sens des flèches (=>).

Un module de pilotage et de traitement 35, telle une carte électronique, est relié électriquement aux capteurs 31, 32 et au débitmètre 33 de manière à récupérer et traiter les mesures opérées par les capteurs 31, 32 et le débitmètre 33. Une source d'énergie, telle une batterie électrique ou une pile, permet d'alimenter le module de pilotage et de traitement 35 en courant électrique.

La Figure 4 schématise une installation d'oxygénothérapie selon l'invention comprenant une source de gaz respiratoire 41, qui est ici une bouteille de gaz, et une interface de distribution de gaz 42 permettant de distribuer le gaz respiratoire à un patient, tel qu'ici des canules nasales, et un dispositif 30 de suivi ou d'observance à débitmètre à oscillation fluidique selon l'invention, tel celui schématisé en Figure 3.

Afin de montrer l'importance du bon positionnement des orifices de mesure 24, 25 selon la présente invention, des essais de simulation ont été réalisés comme expliqué ci-après.

La géométrie du débitmètre choisi (cf. Figure 1) pour les essais de simulation en 2D et les paramètres de simulation sont les suivants :
- modélisation avec le logiciel Ansys Fluent, et maillage en 2D avec Ansys ICEM CFD sur une base de 47881 mailles.
- condition aux limites d'entrée : débit d'entrée fixé.
- condition aux limites à la sortie : débit sortant.
- simulation en régime instationnaire.
- modèle laminaire.

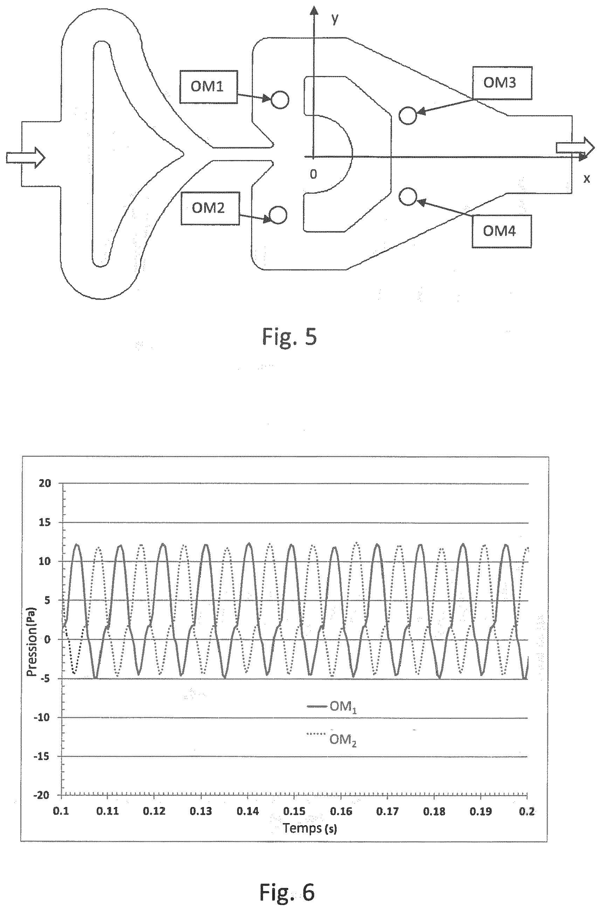

Plusieurs orifices de mesure virtuels OM1 à OM4 ont été positionnés pour évaluer l'impact de leur emplacement/positionnement, en termes d'amplitude et de fréquence du signal de pression associé. On a fait varier le positionnement des orifices de mesure 24, 25 (appelés OM1 à OM4 sur Figure 5) reliés aux microphones ou organes de mesure de pression mesurant la présence ou non d'une dépression.

**Tableau : Différentes positions des axes des orifices de mesure par rapport à l'origine des axes se trouvant au centre du cercle intérieur de la cavité**

| Position des capteurs | x (mm) | y (mm) |
|---|---|---|
| 1 | -1,5 | 2,25 |
| 2 | -1,5 | -2,25 |
| 3 | 3,5 | 2 |
| 4 | 3,5 | -2 |

Après plusieurs essais de positionnement des orifices OM1 à OM4 (cf. Tableau ci-avant), il a été noté que la mesure de pression qui permet de déduire le débit du gaz n'est pas correcte, lorsque la place des prises de mesure acoustique n'est pas choisie avec soin.

En effet, dans le cadre des essais, il a été mis en évidence qu'il convient de positionner les orifices de mesure 24, 25 reliés aux capteurs, symétriquement par rapport au plan P et en respectant une distance d entre les axes des prises de mesure comprise entre 0,5 et 15 mm et ce, pour garantir des mesures de qualité. Les meilleurs résultats sont obtenus pour une distance d entre les axes des prises de mesure comprise entre 1 et 10 mm, préférentiellement entre 1 et 6 mm, typiquement de l'ordre de 3 à 5 mm.

Avantageusement leur position est choisie de préférence dans la zone Z3 de la Figure 1, qui sont délimitées notamment par la droite perpendiculaire en entrée de la chambre d'oscillation 2 au plan de symétrie P, et la droite correspondant à l'intersection entre le plan de symétrie et la cavité semi-cylindrique de l'organe de reflux 21.

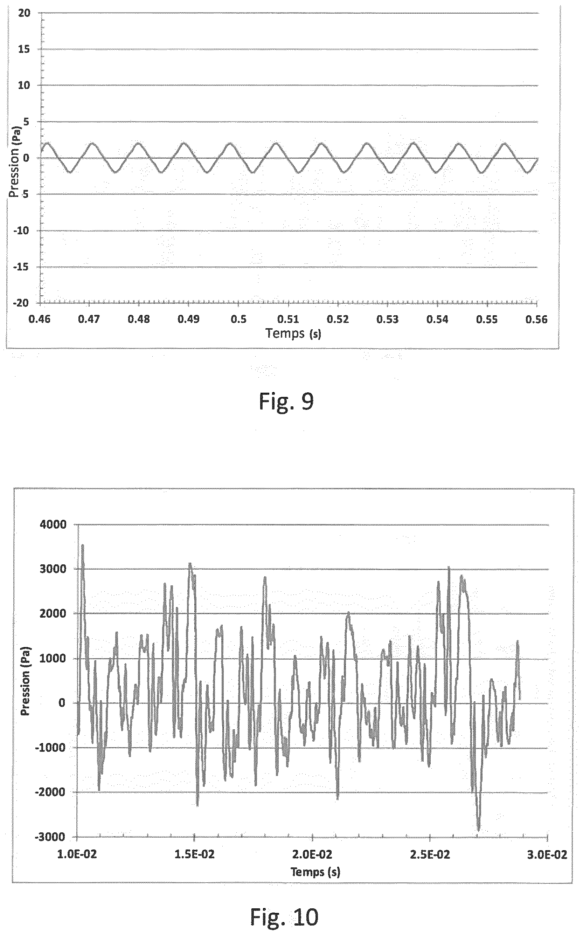

Ces résultats sont représentés sur les Figures 6 à 12. Le débit d'oxygène de 5 L/min pour les Figures 6 à 9, et de 4 L/min pour les Figures 10 à 12.

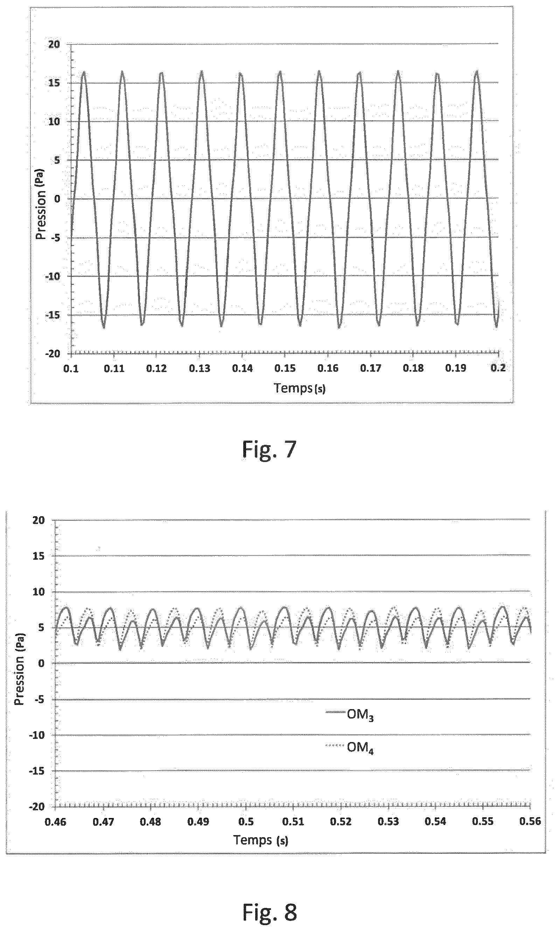

La Figure 6 représente les signaux de pression (en Pa) en fonction du temps pour les deux orifices de mesure OM1 et OM2, alors que la Figure 7 représente le signal de différence de pression (en Pa) entre les deux orifices de mesure OM1 et OM2 en fonction du temps (en sec).

De même, la Figure 8 représente les signaux de pression (en Pa) en fonction du temps (en sec) pour les deux orifices de mesure OM3 et OM4, alors que la Figure 9 représente le signal de différence de pression (en Pa) entre les deux orifices de mesure OM3 et OM4 en fonction du temps (sec).

Sur la figure 6, on voit le signal de pression en fonction du temps (sec) vu par les capteurs (microphones) reliés aux deux orifices de mesure OM1 et OM2 placés dans la zone Z3 et symétriquement par rapport au plan P. L'emplacement exact des deux orifices de mesure est défini dans le tableau ci-avant. L'amplitude de variation du signal de pression (différence entre les valeurs maximum et le minimum) en fonction du temps pour les 2 deux orifices de mesure OM1 et OM2 est de 17 Pa. Il est préférable de faire la différence des signaux des 2 capteurs (Figure 7). En effet, cela permet de presque doubler l'amplitude de variation de pression (on passe de 17 Pa à 34 Pa) mais aussi de limiter le bruit qui peut apparaître sur les signaux de pression (bruit dû à la variation de pression liée à la fréquence respiratoire par exemple).

Sur la Figure 8, on voit le signal de pression en fonction du temps vu par les deux orifices de mesure OM3 et OM4 placés en dehors la zone Z3. On remarque qu'ils sont à la même phase de variation, alors que dans le cas des deux orifices de mesure OM1 et OM2, ils étaient en opposition de phase. L'amplitude de variation de pression pour les 2 deux orifices de mesure est de seulement 5 Pa, donc nettement plus faible que la valeur de 17 Pa pour les deux orifices de mesure OM1 et OM2. La différence des 2 signaux de pression pour ces 2 deux orifices de mesure (voir Figure 9) reste presque à la même amplitude de 5 Pa, alors que l'amplitude de la différence de pression pour les deux orifices de mesure OM1 et OM2 est nettement plus élevée à 34 Pa.

En conclusion, il faut placer les deux orifices de mesure reliés aux capteurs de pression ou microphones dans la zone Z3 mais surtout symétriquement par rapport au plan P et en respectant une distance d entre eux comprise entre 0,5 et 10 mm, de préférence de l'ordre de 1,5 à 6 mm.

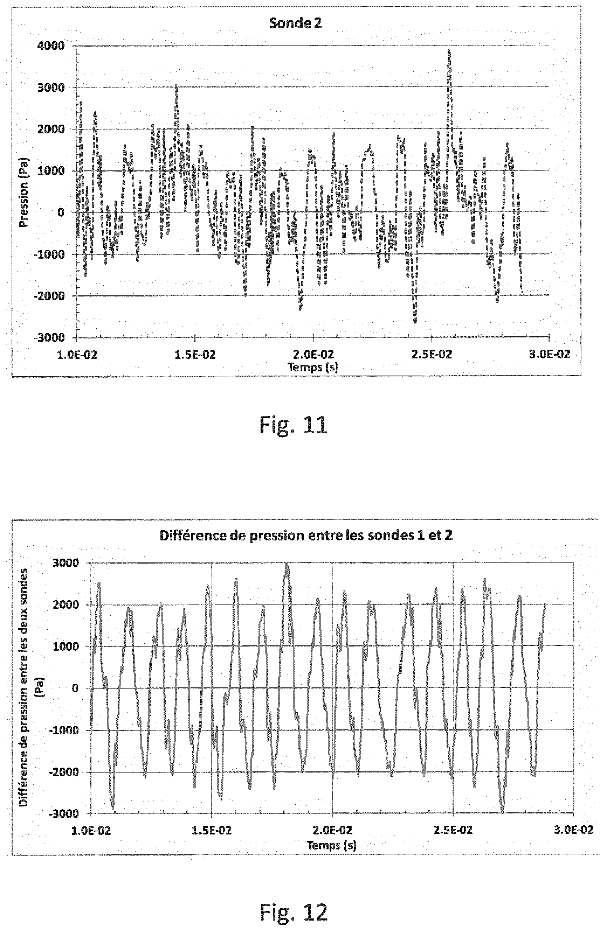

Pour montrer l'importance de faire la différence des signaux des 2 capteurs de pression ou microphones afin d'extraire la fréquence d'oscillation, les Figures 10 et 11 montrent les signaux de pression (en Pa) captés séparément par les orifices de mesure OM1 (aussi appelé « Sonde 1 ») et OM2 (aussi appelé « Sonde 2 ») pour un débit d'oxygène de 4 L/min, ainsi que la différence de ces 2 signaux (Figure 12).

Si on examine séparément le signal de pression relevés par les Sondes 1 et 2 (i.e. les orifices de mesure OM1 et OM2), il est plus difficile d'extraire la fréquence d'oscillation, alors que celle-ci est bien plus visible sur la différence des signaux. D'où la nécessité de faire la différence des signaux de pression et surtout à haut débit, i.e. supérieur à 1 L/min. D'autre part, cela permet d'éliminer dans le cas où existent le bruit électronique sur les 2 capteurs et le bruit acoustique ambiant, ainsi que les variations de pression induites par la fréquence respiratoire du patient. En effet, tous ces bruits (électronique, acoustique, respiration du patient) perturbent les signaux de pression des 2 capteurs de la même façon. Il est donc préférable de les éliminer en opérant une différence entre les signaux de pression.

Le débitmètre à oscillation fluidique de l'invention est particulièrement bien adapté à une utilisation au sein d'un dispositif de suivi ou d'observance d'un traitement d'oxygénothérapie d'un patient à domicile, ledit dispositif de suivi ou d'observance étant relié, d'une part, à une source de gaz respiratoire et, d'autre part, à une interface de distribution de gaz, tel un masque respiratoire, une canule nasale ou analogue, servant à fournir du gaz respiratoire, typiquement de l'oxygène gazeux, au patient.

## Revendications

1. Débitmètre à oscillation fluidique comprenant :
- une chambre de stabilisation (1) comprenant un élément stabilisateur de flux (11),
- une chambre d'oscillation (2) comprenant un élément à reflux (21) configuré pour créer au moins un tourbillon gazeux oscillant dans la chambre d'oscillation (2), ledit élément à reflux (21) étant compris entre deux parois parallèles (28, 29) délimitant la chambre d'oscillation (2),
- un conduit de liaison (3) reliant fluidiquement la chambre de stabilisation (1) à la chambre d'oscillation (2), et
- un plan de symétrie (P) séparant le conduit de liaison (3), la chambre de stabilisation (1), l'élément stabilisateur de flux (11), la chambre d'oscillation fluidique (2) et l'élément à reflux (21) en deux parties égales et symétriques par rapport audit plan de symétrie (P),
**caractérisé en ce que** :
- l'une desdites deux parois parallèles (28, 29) délimitant la chambre d'oscillation (2) comprend deux orifices de mesure (24, 25) agencés symétriquement par rapport au plan de symétrie (P) et sur un axe perpendiculaire au plan de symétrie (P), et séparés l'un de l'autre d'une distance (d) comprise entre 0.5 mm et 15 mm, et
- le conduit de liaison (3) a une section rectangulaire de largeur lₒ et de hauteur hₒ telles que : 6,5.lₒ ≥ hₒ ≥ 3.lₒ.

2. Débitmètre selon la revendication précédente, **caractérisé en ce que** la largeur lₒ et la hauteur hₒ du conduit de liaison (3) sont telles que :
- hₒ ≥ 3,1.lₒ, de préférence hₒ ≥ 3,5.lₒ, et/ou
- 6.lₒ ≥ hₒ, de préférence 5,5.lₒ ≥ hₒ.

3. Débitmètre selon l'une des revendications précédentes, **caractérisé en ce que** le conduit de liaison (3) de section rectangulaire a une longueur Lₒ telle que : 2.lₒ ≤ Lₒ ≤ 10.lₒ.

4. Débitmètre selon l'une des revendications précédentes, **caractérisé en ce que** les deux orifices de mesure (24, 25) sont séparés d'une distance (d) comprise entre 0,5 mm et 10 mm, de préférence entre 1 mm et 6 mm.

5. Débitmètre selon l'une des revendications précédentes, **caractérisé en ce que** l'élément stabilisateur de flux (11) a une section de forme générale triangulaire et/ou l'élément à reflux (21) comprend une partie (22) de section semi-cylindrique agencée face au conduit de liaison (3).

6. Débitmètre selon l'une des revendications précédentes, **caractérisé en ce que** :
- la chambre de stabilisation (1) comprenant un premier orifice d'entrée (12) et un premier orifice de sortie (13) agencés sur le plan de symétrie (P) et
- la chambre d'oscillation (2) comprenant un second orifice d'entrée (23) et un second orifice de sortie (26) agencés sur le plan de symétrie (P),
- le conduit de liaison (3) reliant fluidiquement le premier orifice de sortie (13) de la chambre de stabilisation (1) au second orifice d'entrée (23) de la chambre d'oscillation (2).

7. Débitmètre selon l'une des revendications précédentes, **caractérisé en ce qu'**il comprend en outre un ou plusieurs capteurs de pression ou microphones raccordés auxdits deux orifices de mesure (24, 25) de manière à permettre de mesurer la pression dans la chambre d'oscillation (2).

8. Débitmètre selon l'une des revendications précédentes, **caractérisé en ce que** les deux orifices de mesure (24, 25) sont fermés par une membrane fluidiquement étanche.

9. Débitmètre selon l'une des revendications précédentes, **caractérisé en ce qu'**un canal d'entrée (4) est relié fluidiquement au premier orifice d'entrée (12) de la chambre de stabilisation (1).

10. Débitmètre selon l'une des revendications précédentes, **caractérisé en ce qu'**il comprend en outre un boitier au sein duquel sont agencés le conduit de liaison (3), la chambre de stabilisation (1), l'élément stabilisateur de flux (11), la chambre d'oscillation fluidique (2), l'élément à reflux (21) et le ou les capteurs de pression ou microphones.

11. Débitmètre selon l'une des revendications précédentes, **caractérisé en ce que** l'élément stabilisateur de flux (11) est espacé de la paroi périphérique (14) de la chambre de stabilisation (1) de manière à créer des passages (15) pour le gaz autour dudit élément stabilisateur de flux (11).

12. Dispositif de suivi ou d'observance d'un traitement d'oxygénothérapie comprenant un débitmètre à oscillation fluidique selon l'une des revendications précédentes.

13. Installation d'oxygénothérapie comprenant :
- une source de gaz respiratoire,
- une interface de distribution de gaz permettant de distribuer le gaz respiratoire à un patient, et
- un dispositif de suivi ou d'observance à débitmètre à oscillation fluidique selon la revendication 12.
